# EUROPEAN PATENT SPECIFICATION

(11) **EP 4 380 655 B1**
(45) Date of publication and mention of the grant of the patent: **21.05.2025**
(21) Application number: 22782567.6
(22) Date of filing: 03.08.2022
(51) Int. Cl.: A61M 11/00, A61M 11/06, A61M 16/08, A61M 16/10, A61M 16/12, A61M 16/16, A61M 25/00

(54) **RESPIRATORY DEVICE AND A RESPIRATORY SET INTENDED THEREFOR**
BEATMUNGSVORRICHTUNG UND DAZU BESTIMMTES BEATMUNGSSET
DISPOSITIF RESPIRATOIRE ET ENSEMBLE RESPIRATOIRE DESTINÉ À CET EFFET

(30) Priority: 03.08.2021 NL 2028916
(43) Date of publication of application: 12.06.2024
(73) Proprietor: Medspray Humidification B.V., 7521 PV Enschede (NL); Medspray B.V., 7521 PV Enschede (NL)
(72) Inventor: VAN RIJN, Cornelis Johannes Maria, 7521 PV Enschede (NL); VAN EGMOND, Henri Joseph, 7521 PV Enschede (NL); VAN DER VEGTE, Stefanus Maria, 7521 PV Enschede (NL); HUIJGEN, Tom Vincent, 7521 PV Enschede (NL); KROON, Eric Jacobus Cornelis, 7521 PV Enschede (NL)
(74) Representative: Jilderda, Anne Ayolt
(86) International application number: PCT/IB2022/057215
(87) International publication number: WO 2023/012696

(56) References cited:
- WO-A1-2019/222159
- WO-A1-2020/197414
- WO-A2-2009/049909

## Description

The present invention relates to a respiratory device, comprising artificial respiration means for generating and maintaining an oxygen-containing respiratory gas flow; a respiratory tube in which a respiratory channel extends for the purpose of carrying the respiratory gas flow to an outlet for supply of the respiratory gas flow for the respiratory system of a patient, which respiratory tube is coupled or at least couplable to the respiratory means; humidification means coupled or at least couplable to the respiratory tube for the purpose of humidifying the respiratory gas flow in the respiratory channel with a humidification fluid; and a humidification fluid source which is coupled or at least couplable via a fluid conduit to the humidification means for the purpose of providing the humidification fluid thereto.

Such a device is applied particularly in hospitals and other healthcare facilities, and more specifically in an operating room and/or an intensive care unit for thereby artificially supporting or even taking over completely a patient's breathing. A respiratory device used for this purpose usually comprises an advanced respiratory station with a ventilator whereby an oxygen-rich inspiratory airflow is maintained, which is guided via a respiratory tube to the patient. Such a station often also comprises a return conduit for receiving the expiratory air from the patient and ridding it of carbon dioxide. Once enriched with oxygen, this airflow can then be fed back to the patient again as inspiratory air. Such a respiratory station usually has a plurality of sensors and an extensive user interface with screen whereby the composition of the two airflows is accurately monitored and controlled.

In some cases it is however also desirable to artificially support and maintain a victim's breathing in ambulatory situations, such as for instance at the location of the person involved or in the case of accidents and calamities. In these cases the respiratory means usually comprise a compressed air or oxygen cylinder with pressure controller from which is taken an oxygen-rich inspiratory airflow, which is guided via a respiratory tube with nozzle to the victim.

Although the person involved can thus be fed an oxygen-rich gas flow in order to thereby support or even fully take over their own breathing, it has been found in practice that the administration of dry air can result in harmful dehydration of the person involved. This in turn results in serious health risks and symptoms. In order to prevent these it is important to humidify the inspiratory gas flow before guiding the gas flow into the respiratory system of the person involved.

A known humidification device is for instance known from American patent application US 2010/0242963 in name of the current market leader in the field of respiratory equipment. This device provides an evaporator device in the respiratory tube. The evaporator device comprises a water bath with a water supply and heating element which are in open communication with an evaporation chamber. The evaporation chamber comprises an inlet for the respiratory channel and an outlet, and thus allows the respiratory gas flow to flow through the evaporation chamber and here absorb water vapour escaping from the water bath.

Although the moisture content of the inspiratory airflow can thus be increased, such evaporation-based humidification devices have drawbacks in practice. Firstly, the heating of the water bath requires some time, and the device thereby has a relatively long start-up phase. In addition, the humidification of the inspiratory airflow in such a device is inevitably limited to the maximum relative air humidity at the given temperature of the airflow. At this level the air is saturated and unable to absorb more water vapour. The risk is therefore high that a part of the introduced humidification fluid will precipitate as condensation in the respiratory tube, whereby the content which has actually reached the respiratory system of the patient is unknown.

A humidification device based on a different humidification mechanism is for instance known from International patent application WO 2008/117265. The humidification means of this device comprise a water bath which is in contact with a piezoelectric vibrator element. The vibration generated thereby disturbs the water surface and thus creates an aerosol of water droplets which is delivered to the inspiratory air. A part of the fluid thus introduced into the airflow will evaporate and a remaining part will be entrained in the form of liquid droplets. WO2019222159 discloses an atomizer and heating means in breathing tube. The heating means is downstream the atomizer.

An advantage of such a piezoelectric humidifier is that the maximum water assimilation of the inspiratory airflow is thus not limited by the maximum relative air humidity at the prevailing temperature, but that a proportion of moisture in liquid form can also be absorbed and entrained in the water droplets. A drawback is however that the piezoelectric aerosol generator requires its own electric power supply, control and connections, which is not only impractical but moreover susceptible to malfunction. In this known device the aerosol generator is also an integral part of the overall device, and must therefore always be thoroughly sterilised before a subsequent patient can be treated. What's more, the humidification with such a humidifier is not very accurate in that it is difficult to control exactly how much moisture is delivered in the airflow and actually entrained therein. And this device also has the risk that a part of the evaporated humidification fluid will precipitate as condensation downstream in the respiratory tube instead of actually reaching the respiratory system of the patient.

The present invention has for its object, among others, to provide a respiratory device whereby a dosage of a humidification fluid which is controlled exceptionally precisely and is not limited by a relative air humidity can be delivered to a respiratory gas flow. In a further aspect the present invention has for its object, among others, to provide a humidification device with humidification means which are at least largely exchangeable and replaceable therefrom and can thus be provided as disposable item.

In order to achieve the stated object a respiratory device of the type described in the preamble has the feature according to the invention that the humidification means comprise an atomizer which is able and configured to form a fine mist when the humidification fluid is excited and to deliver the fine mist on an outlet side; that the atomizer is in open communication on the outlet side with the respiratory channel in the respiratory tube; that heating means are provided which enter into heat-exchanging contact with the respiratory gas flow upstream of the atomizer in order to dissipate heat thereto, and that at least the atomizer and the heating means are controlled by electronic control means configured to mutually adapt a heat dissipation by the heating means in the respiratory gas flow and a downstream delivery of the fine mist by the atomizer such that said heat dissipation suffices to make the mist formed by the atomizer transition substantially wholly, and particularly wholly, into a vapour phase when delivered by the atomizer. The humidification fluid which transitions from the liquid aerosol phase into the gas phase removes energy from the gas flow, which will thereby cool down. In order to compensate for this, the heating means already provide sufficient heat to the gas flow upstream to already compensate for this beforehand, so that the gas flow remains above a dew point thereof at all times and condensation is counteracted. The control means can here comprise a control device adapted thereto and provided separately, or can be integrated with one or more remaining parts of the control of the respiratory device.

It is important that the device according to the invention allows for introduction into the gas flow of a precisely determined, absolute amount of moisture which is not limited by a maximum absorption capacity for water vapour of the gas flow at room temperature. The (pre)heating of the gas flow ensures that the introduced aerosol can transition fully into the gas phase and in this form is entrained by the gas flow. Not only is a greater moisture delivery thus possible, it is moreover conducive to the whole dose of humidification fluid introduced in this way actually reaching the respiratory system of the patient.

It is important that the amount of fluid which is introduced to humidify the respiratory gas flow is received therein as much as possible as vapour, and then remains in this aggregation state. In order to avoid overdosing, which could otherwise result in condensation forming, a preferred embodiment of the respiratory device according to the invention therefore has the feature that humidification fluid is fed to the atomizer by electronically controllable dosing means, which dosing means are controlled by the control means.

The amount of humidification fluid to be introduced can then be precisely adapted to the maximum absorption capacity in the respiratory gas flow. The dosing means applied for this purpose can be of diverse nature. In a particular embodiment the respiratory device has for this purpose the feature that the dosing means comprise an electronically controllable metering pump which feeds the atomizer. An advantage of a metering pump is that it can often be proportionally controlled so that a precisely measured quantity of humidification fluid is supplied to the atomizer at the intended operating pressure. In an alternative or further particular embodiment the respiratory device according to the invention is characterized in that the dosing means comprise an electronically controllable valve device between the atomizer and the humidification fluid source. Such a valve device, such as for instance an electronically switchable valve, can for instance be applied individually for the atomizer so that the atomizer can be controlled individually. The valve device can here not only close or open completely, but can also be controlled intermittently for the purpose of a proportional control of the atomizer controlled thereby.

The respiratory device according to the invention allows for an absolute amount of dosed fluid to be added to the respiratory gas flow, on condition that this gas flow is not (yet) saturated. The maximum moisture content until saturation depends on the gas temperature and thus provides an upper limit to the maximum permissible amount of moisture that can be introduced. A further preferred embodiment of the respiratory device according to the invention in this respect provides a real-time control of the maximum moisture supply and is for this purpose characterized in that upstream of the heating means at least one sensor from a group of an air humidity sensor, a temperature sensor and a flow sensor is in operative contact with the respiratory gas flow, which at least one sensor is coupled to the control means for generating a control value thereto. The one or more control values which are thus exchanged with the control means enable the control means to adapt a dosage of the humidification fluid to the maximum content determined therefrom by controlling the atomizer accordingly and/or increasing the maximum quantity by having the heating means generate more heat.

Besides upstream or at the position of the humidification means, condensation can also still occur later, downstream, for the first time if the respiratory gas flow were to become saturated. A further preferred embodiment of the respiratory device according to the invention therefore has the feature that downstream of the humidification means at least one sensor from a group of an air humidity sensor, a temperature sensor and a flow sensor is in operative contact with the respiratory gas flow, which at least one sensor is coupled to the control means for generating a control value thereto. A value of the relative air humidity at the position of the sensor is obtained by means of the air humidity sensor. This value must remain below 100%. When this value is approached, the control means can intervene by drawing upon the heating means in order to increase the temperature of the respiratory gas flow or limit the moisture delivery by the humidification means.

In order to keep an influence of a possible cooling of the respiratory gas flow downstream of the humidification means under control and optionally compensate for this influence, a further preferred embodiment of the respiratory device according to the invention has the feature that close to the outlet at least one sensor from a group of an air humidity sensor, a temperature sensor and a flow sensor is in operative contact with the respiratory gas flow, which at least one sensor is coupled to the control means for generating a control value thereto.

There is ultimately a limit to the maximum permissible amount of heat which can be dissipated to the respiratory gas flow and which is controlled by patient safety. With a view thereto, a further preferred embodiment of the respiratory device according to the invention has the feature that the control means control the heating means and the humidification means in mutual relation such that the respiratory gas flow has a temperature close to the outlet which is above a dew point of the respiratory gas flow and below a maximum temperature which is still safe for the patient's respiratory system, particularly a temperature that is or is close to body temperature.

In this embodiment the focus is on a safe temperature for the patient as provided in the respiratory gas flow at the outlet. Controlling the heating means and humidification means such that this temperature remains above the dew point in situ and also remain safe for the patient enables an optimal moisture content to be maintained in the respiratory gas flow. This is based on the insight that downstream of the heating means the respiratory gas flow will usually only cool down, and that the lowest temperature, therefore with the greatest risk of condensation, will occur at the outlet. By thus removing or at least controlling this risk the danger of condensation forming will thereby also be removed elsewhere in the device.

A further preferred embodiment of the respiratory device according to the invention has the feature that the humidification means comprise a Rayleigh type atomizer, comprising an atomizer body with a spray wall in which at least one microscopic spray opening extends over a whole thickness thereof, which at least one microscopic spray opening provides a passage between the inlet side and the outlet side of the atomizer and receives the humidification fluid under an increased operating pressure on the inlet side in order to dispense a jet thereof of mutually at least substantially identical successive liquid droplets of at least substantially a determined first size on the outlet side, and that pressure means are provided which impose the increased operating pressure on the humidification fluid and guide the humidification fluid to the atomizer under the increased operating pressure. The humidification means thus comprises an aerosol generator in the form of an atomizer which, fed the humidification fluid under increased pressure, forms a vapour from the humidification fluid. The energy required for this is derived by the atomizer from the fluid pressure. No further electronic control nor electronic connection for the atomizer is per se required for this purpose. The latter in particular provides a great practical advantage and allows the humidification means to be embodied to significant extent as easily exchangeable disposable item.

This embodiment is moreover based on the insight that if use is made of Rayleigh atomization, the size of the droplets is mainly determined and imposed by the operational cross-section of the microscopic spray opening. This operational cross-section can be defined with great precision, for instance photolithographically, and so can thereby thus the size of the droplets which result therefrom and which will usually be in the order of twice to three times greater. This means that the formed aerosol will in that case comprise substantially only almost identical droplets of the same microscopic size. The fluid flow rate supplied via the spray opening can thus be translated into a corresponding number of liquid droplets of this size. The behaviour thereof in the respiratory gas flow, particularly the transition to the vapour phase, is thus uniform and readily predictable. The microscopic size of the droplets, among other things, is moreover conducive to the evaporation taking place almost instantaneously before the mist is able to reach the wall of the respiratory tube. The moisture content of the gas flow imposed thereby can thus be controlled within particularly precisely determined limits.

A further preferred embodiment of the device according to the invention has the feature that the humidification means comprise at least one similar further atomizer of the Rayleigh type with at least one further microscopic spray opening which receives the humidification fluid under an increased operating pressure on the inlet side in order to dispense a jet thereof of mutually at least substantially identical successive liquid droplets of at least substantially a determined further size on the outlet side. Because more atomizers thus debouch parallel to each other in the respiratory channel, an overall dispensing capacity can be increased or a spray pattern otherwise fine-tuned. Different atomizers can be combined with each other and be drawn upon individually as needed.

In this latter respect a particular embodiment of the device has the feature that the humidification means comprise at least one similar further atomizer of the Rayleigh type with at least one further microscopic spray opening which receives the humidification fluid under an increased operating pressure on the inlet side in order to dispense a jet thereof of mutually at least substantially identical successive liquid droplets on the outlet side, which at least one further spray opening differs in number thereof from the at least one first microscopic spray opening of the first atomizer. By varying the number of spray channels per atomizer a variable fluid flow rate can thus for instance be opted for by specifically drawing upon or not drawing upon one or more atomizers.

In a further embodiment the device according to the invention is characterized in that each of the at least one spray opening of the first atomizer has a first fluid-transporting cross-section, that each of the at least one spray opening of the further atomizer has a further fluid-transporting cross-section, which further fluid-transporting cross-section differs from the first fluid-transporting cross-section. A vapour of finer or thicker droplets can thus be generated as desired by selective operation of one or more atomizers.

For the humidification fluid use can simply be made of water or a physiological saline. The many microscopic droplets together provide a relatively large evaporating surface. Due to the evaporation the respiratory gas flow will become rapidly saturated. The relative humidity is then 100%. A remainder of microscopic aerosol can however be entrained in liquid form by the respiratory gas flow, which can thus become oversaturated and can contain more moisture than was possible solely through evaporation. The extent to which this is the case is imposed in precise manner by adapting the provided fluid flow rate to the volume of the gas flow. All in all, the respiratory gas flow can thus be set particularly efficiently and effectively to a precisely determined moisture content.

In a practical embodiment the device is here characterized in that the respiratory tube is bounded at least locally by a wall and comprises in the wall a wall opening in which an atomizer is fittingly received. One or more atomizers are thus integrated directly in the wall of the respiratory tube and thus debouch directly in the respiratory channel. The provided fluid flow rate thus flows directly into the respiratory gas flow as microscopic aerosol without losses. For a seamless, liquid-tight and gastight seal in the wall of the respiratory tube use can advantageously be made of a preferred embodiment of the device according to the invention which is characterized in that the atomizer is placed into the wall opening via a fitting piece. The atomizer itself can thus be a more or less standard product which is internally received fittingly by the fitting piece. The fitting piece forms an adapter and is in turn externally adapted to the material and/or the dimensions of the wall opening and optionally provided with a sealing intermediate gasket.

In a further particular embodiment the device according to the invention has the feature that the atomizer and the fitting piece together form an assembly which fits releasably in the wall opening as stand-alone component. The fitting piece and the atomizer are in that case advantageously assembled into such a stand-alone component beforehand. Additional functionality can optionally be imparted thereto. In this respect a further particular embodiment of the device according to the invention has the feature that the fitting piece comprises upstream of the atomizer a microscopic preliminary filter, particularly a microbial preliminary filter with maximal microscopic meshes smaller than 3 micrometres. Such a preliminary filter prevents any blockage and thereby disruption of the atomizer, although it can also serve as sterile barrier. It is particularly if the preliminary filter has a largest passage smaller than 0.5 to 3 microns, which is usually too small for microbes such as fungi and bacteria, that an effective microbial barrier can be formed thereby. A microbial invasion of the respiratory channel from the humidification means can thus be prevented.

In addition to humidifying the respiratory gas flow, the setup of the respiratory device according to the invention also provides an option of introducing an active substance therein. For this purpose a further particular embodiment of the device according to the invention has the feature that at least one further atomizer is provided, which is fed by a fluid source of a further fluid and is able and configured to form a further fine mist from the further fluid and deliver it in the respiratory channel, wherein the further fluid contains a pharmaceutically active substance. A preferred embodiment of the respiratory device has the feature here according to the invention that the further atomizer and the humidification means are controlled and regulated in mutual relation by shared control means. This particularly enables the control means of the respiratory device to adapt a humidification in the respiratory gas flow to an administration, whether momentary or not, of a medication by means of the further atomizer in order to allow both the humidification and the medicinal administration to take place optimally.

A pharmaceutically active fluid can thus be introduced just as the humidification fluid into the respiratory gas flow. The control thereof can here be performed by the same control means which are provided for the humidification means and/or the respiratory means, but a separate, specifically adapted control device, which is for instance integrated with a metering pump, can also be provided for this purpose.

The medication can thus be administered fully automatically and the dispensing thereof can be adapted particularly to the humidification by the humidification means and/or the gas flow maintained by the respiratory means, or vice versa. It is thus particularly possible in the administration of the medication to take into consideration information about the actual gas flow as delivered by the respiratory means or as registered by a flow sensor in the gas flow. Using this information, the administration of the medication can be adapted to a respiration pattern of the patient, which results in a significant increase in the efficiency of the consumption of the medication and the effectiveness of the administration by distributing the medication only during inspiration.

Introduction of the medication preferably takes place downstream of the heating means. By evaporating the pharmaceutical fluid, whether wholly or not, the droplet or particle size of the medicinal aerosol can be optimized so that as little aerosol as possible is lost in the respiratory circuit. This is because smaller, partially evaporated droplets will be entrained further and will precipitate in the respiratory channel to lesser extent. By controlling the degree of evaporation an administration of the medication can thus take place more efficiently.

If desired, the pharmaceutical can otherwise also be carried to the relevant atomizer under an adequate operating pressure manually and be delivered in the airflow and administered to the patient. The pharmaceutically active substance can be of diverse nature and thus, with a view to a therapeutic effect to be achieved thereby, be administered in respiratory manner, for instance an anaesthetic such as Lidocaine or Xylocaine, an anti-inflammatory or other type of therapeutic medicine or agent.

A particularly direct heating of the respiratory gas flow can be brought about with heating means comprising a heating element which enters into direct contact with the gas flow for heating, for instance in that the gas flow is guided through it or over it. The preheating of the respiratory gas flow upstream of the humidification means can be performed both in-line and parallel to the respiratory channel. In this latter respect a particular embodiment of the respiratory device according to the invention has the feature that an air chamber debouches at the position of the atomizer in the respiratory channel and that the air chamber is provided with heating means for bringing a volume of air held therein to an increased temperature, and that provided between the air chamber and the respiratory channel are valve means which are connected operatively to the control means for letting in the volume of air of increased temperature at least substantially simultaneously to the fine mist.

With a view to a heating of the respiratory flow the respiratory tube can also be provided in the wall with electrically energizable heating means intended and configured to enter into heat-exchanging contact with the respiratory gas flow. The heating means are able to increase a temperature of the gas flow close to the wall and so counteract formation of condensation thereon. A practical embodiment is characterized here in that the heating means comprise at least one heating element in the wall of the respiratory tube, particularly a filament incorporated in the wall of the respiratory tube over a length. Energy can thus particularly be supplied to the wall downstream of the humidification means, which energy will result in a further temperature increase of the gas flow downstream of the atomizer(s) so that vapour entrained in the gas flow will not condense after all, whether partially or not.

For an accurate monitoring and possible control of the humidification of the respiratory gas flow a preferred embodiment of the device is characterized in that the control means in the respiratory device are operatively coupled to one or more of electronically energizable valves, electronically energizable heating elements and electronic sensors, such as temperature sensors, moisture sensors, gas and liquid flow rate sensors and also the ventilator of the respiratory means. An adequate feedback of various sensor signals to the control means enable these means to correctly control and fine-tune the different actors so that a predetermined moisture content in the respiratory gas flow will be ensured. For this purpose the control means are able and configured to draw upon, whether intermittently or not, one or more of the atomizers and/or to control the respiratory means and/or possible heating means in order to adapt a respiratory gas flow rate in the respiratory gas conduit and a desired degree of humidification of the respiratory gas flow to each other.

The humidification fluid can per se be drawn from various sources, wherein a sufficient increased operating pressure, typically in the order of 5 to 30 bar, is imposed thereon. A particularly practical setup in this respect is applied in a further embodiment of the device according to the invention, which is characterized in that the dosing means comprise a container with a container volume in which a piston is movable in close-fitting manner along a first stroke and a second stroke, wherein the humidification fluid is taken from the humidification fluid source in the first stroke and is forced via the fluid conduit to the atomizer under the increased pressure in the second stroke. The fluid reservoir and the atomizer are here uncoupled from each other by the dosing device, which communicates with the fluid reservoir with the first stroke in order to take the fluid therefrom and carries the taken fluid to the atomizer under sufficient operating pressure with the second stroke.

The dosing device can bring about this uncoupling without having to be uncoupled for this purpose. For this purpose a further particular embodiment of the device according to the invention has the feature that the container comprises a combined inlet and outlet, wherein the first stroke and the second stroke comprise a mutually opposing inward and outward stroke of the piston, and wherein the combined inlet and outlet is coupled via non-return valves with opposite orientation to both respectively the humidification fluid source and the fluid conduit. The non-return valves thus provide for the correct fluid flows with opposite orientation to and from the dosing device.

The piston can here be manipulated manually in order to impart the first and second stroke thereto. Nevertheless, a preferred embodiment of the device according to the invention has the feature that the container is provided with an electronically energizable actuator, particularly a linear actuator, which is coupled operatively to the piston and is controlled by the control means. Such a fully energized, for instance motorized, setup allows a particularly precise delivery and pressure build-up of the humidification fluid and thereby unburdens those working therewith.

No particular requirements are made per se of the container with the container volume, other than that the piston can perform the described first and second stroke therein while taking up and supplying the fluid. A particular embodiment of the device therefore has the feature that the container comprises a reservoir of a common clinical syringe. A commonplace syringe cylinder which is freely and widely available can thus be applied as disposable part for the container. This provides an economically particularly advantageous solution. The invention therefore also relates to a respiratory device characterized in that the container together with the atomizer form part of a releasable disposable assembly, wherein the container is coupled to the atomizer via a fluid tube. Said assembly can be supplied as respiratory set which can be used once or at least should be used once and which comprises the container in addition to a part of the liquid-carrying system of the device, including the atomizer(s) and connecting fluid conduits.

The invention also relates to a respiratory set of disposable components for use with the above-described respiratory device according to the invention, comprising a liquid-carrying system of at least the atomizer and a fluid conduit coupled thereto, optionally including a fluid valve incorporated in the fluid conduit and/or a container coupled thereto for the purpose of providing the humidification fluid. This assembly then forms an exchangeable, disposable component of the device. The parts thereof can for instance each be manufactured from a suitable plastic at relatively low cost.

The invention will be further elucidated hereinbelow with reference to an exemplary embodiment and an accompanying drawing. In the drawing:
- Figure 1: is a schematic representation of a first exemplary embodiment of the respiratory device according to the invention;
- Figure 2: is a schematic representation of a second exemplary embodiment of the respiratory device according to the invention;
- Figure 3: is a schematic representation of a third exemplary embodiment of the respiratory device according to the invention;
- Figure 4: is a fourth exemplary embodiment of the respiratory device according to the invention;
- Figure 5: is a cross-section of a spray chamber with an atomizer of the respiratory device of figure 4;
- Figure 6: is a fifth exemplary embodiment of the respiratory device according to the invention; and
- Figure 7: is a disposable respiratory set for application with the respiratory device according to the invention.

It is otherwise noted here that the figures are purely schematic and not always drawn to (the same) scale. Some dimensions in particular may be exaggerated to greater or lesser extent for the sake of clarity. Corresponding parts are designated in the figures with the same reference numeral.

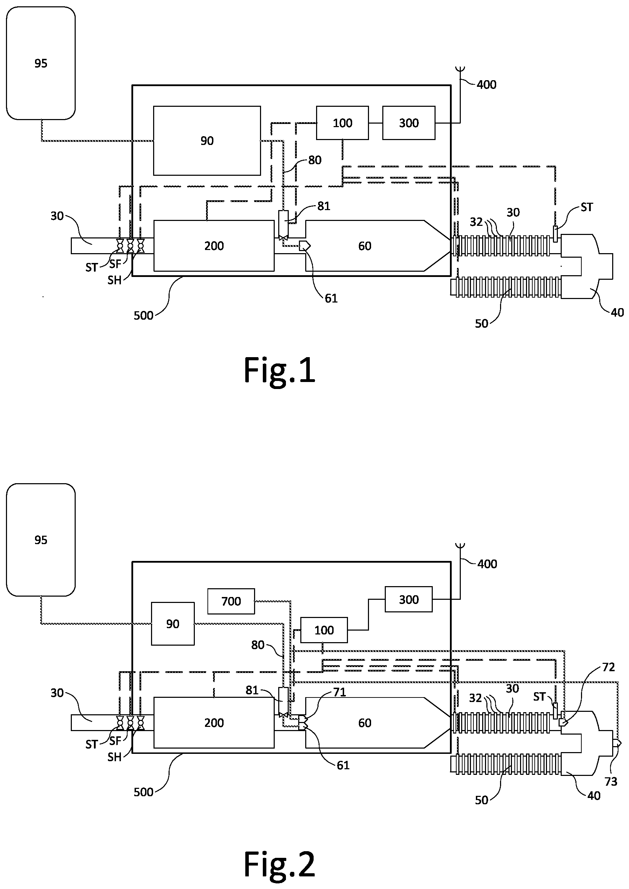

Figure 1 shows schematically a first embodiment of the respiratory device according to the invention. The device comprises a respiratory tube 30 which can be coupled to an outlet of common respiratory means with a ventilator whereby a respiratory gas flow is generated and maintained, which gas flow will also be referred to hereinafter as respiratory airflow or simply airflow. The respiratory airflow is guided to the patient via a respiratory channel extending longitudinally in the respiratory tube.

The respiratory tube 30 is provided with electronically controllable humidification means in order to be able to increase a moisture content in the airflow in precise manner, if desired. The humidification means comprise an atomizer 61 which debouches directly into a spray chamber 60 through which the respiratory channel runs. The atomizer 61 is provided on an inlet side with an electronically controllable shut-off valve 81 which is controlled by electronic, arithmetic control means 100 provided for this purpose. Atomizer 61 is fed a suitable humidification fluid by a pump 90, which fluid is drawn from a reservoir 95 provided for this purpose. The pump 90 produces an operating pressure in the order of between 5 and 30 bar and forces the humidification fluid to atomizer 61 under roughly this pressure. The atomizer is of the Rayleigh type, with one or more microscopic spray openings through which the humidification fluid is guided so it breaks up under the influence of its own kinetics and surface tension into a droplet train of substantially identical microscopic liquid droplets which together form a fine mist. When shut-off valve 81 is opened, this mist is delivered into the respiratory gas flow in respiratory tubes 30 and so adequately humidifies the respiratory gas flow being guided to the patient. Pure water or a medicinal saline is for instance used as humidification fluid.

It is important here to precisely control the moisture content in the respiratory gas flow and to be able to adjust it if necessary. For this purpose a number of sensors is incorporated in the respiratory tube, including a temperature sensor ST, a humidity sensor SH and a flow sensor SF at the inlet of respiratory tube 30. These sensors are coupled to the control means 100 and generate their respective control values thereto. From these data the control means 100 calculate the maximum absolute amount of fluid which can be absorbed by the airflow per unit of time before saturation occurs. This quantity depends on the temperature and the force of the respiratory gas flow and can be added to the already present air humidity detected by the (relative) humidity sensor SH.

For an accurate dosage of the actual humidity content in the airflow as it reaches the patient it is important that the administered quantity of fluid is actually absorbed in the respiratory airflow and does not for example remain behind as liquid on the inner wall of the respiratory tube. The latter results not only in loss of moisture content, but moreover in a lack of clarity regarding the actual percentage of moisture in the air being guided to the patient. In order to prevent or at least counteract this the respiratory device comprises upstream of atomizer 61 heating means 200 which enter into heat-exchanging contact with the respiratory gas flow. The heating means comprise for instance a filament or spiral in the respiratory channel and/or, as they do here, a heating element in the wall of the respiratory tube. Heating means 200 are controlled by the control means 100 and are able to dissipate sufficient heat to the airflow so that the mist formed by atomizer 61 transitions directly into the vapour phase. The mist thereby has no or hardly any chance to precipitate in the form of liquid onto the wall of respiratory tube 30. The heating means 200 and the spray chamber 60 can optionally be accommodated in a shared housing 500 and be incorporated in the respiratory tube as a unit, wherein the inlet sensors ST, SH, SF are then optionally also provided in the same housing 500.

In order to prevent liquid which has thus evaporated almost instantaneously from still condensing onto the wall of the respiratory tube downstream afterward, the wall of the respiratory tube also comprises a heating element 32 downstream, whereby the respiratory gas flow can optionally be heated. This heating element 32 is also coupled to the control means 100 which precisely monitor the temperature of the airflow at the outlet on the basis of a control value generated by a temperature sensor ST provided there. All electronic components are powered by a power source 300 provided for this purpose, which can be both wireless (battery) and can be coupled to a mains electricity 400, as it is here.

From a viewpoint of patient safety, the temperature of the airflow escaping from the device must not exceed a value which is still deemed safe. This value lies around body temperature. As soon as this value is in danger of being exceeded, the system switches off all heating means and a warning is given. Control means 100 particularly control on the basis of the air temperature ST desired at the outlet, the registered air flow rate SF and the initial air humidity SH. The final temperature ST of the airflow ultimately determines the absolute amount of fluid which can be entrained in the airflow without reaching the dew point anywhere in the respiratory device. A maximal humidification is obtained by dispensing this quantity via atomizer 61 and controlling the heating means 200 with sufficient power that the minimum evaporation enthalpy required for this purpose is supplied and dissipated to the airflow. This evaporation heat amounts to 2256 Joule per millilitre of dispensed fluid (water). This must be dissipated into the airflow by the upstream heating means at minimum per unit of time, depending on the airflow.

A second embodiment of the respiratory device is shown schematically in figure 2. The respiratory device is largely identical to that of figure 1, although in this case expanded with one or more further atomizers 71, 72, 73 which debouch in the respiratory channel in order to deliver a pharmaceutically active substance thereto. For this purpose these further atomizers are fed by a medicinal metering pump 700 whereby a fluid containing the pharmaceutical compound is supplied. The metering pump 700 is connected to the control means 100 and is controlled thereby. Although for the further atomizers 71, 72, 73 use can optionally be made of a different type, use is therefor preferably also made of Rayleigh type atomizers owing to their superior operation. The size of the spray opening(s) therein, and thereby the size of the liquid droplets of the mist to be formed thereby, can if desired differ from that of the atomizer or atomizers of the humidification means.

If use is made of a water-based pharmaceutical fluid, this contributes to the overall moisture content of the airflow. The control means 100 take this into account in the control and dosage of the humidification means. Such a further atomizer 71, 72, 73 for dispensing for instance an anaesthetic such as Lidocaine or Xylocaine, an anti-inflammatory or another type of therapeutic medicine or agent can be placed at the atomizer 61 of the humidification means in spray chamber 60, but can also be incorporated downstream in respiratory tube 30 or in a coupling (Wye Piece) 40 at the outlet.

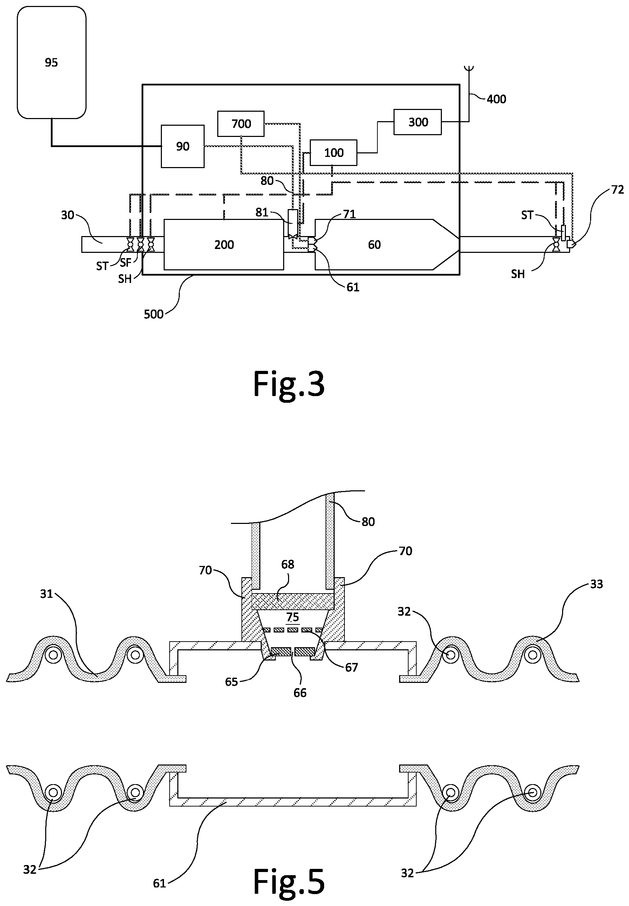

Figure 3 illustrates schematically a third embodiment of the respiratory device according to the invention. This embodiment is largely identical to that of figure 2, albeit that in this case the outgoing respiratory tube 30 comprises only a thermally insulated tube, without additional heating means and without expiratory tube. This embodiment is particularly suitable for respiration at a high flow rate, wherein the airflow has a relatively high rate of flow. In this case the humidification means 60, 61 are also expanded with further atomizers 71, 72 for the purpose of optional administration of the pharmaceutical. A sensor SH which records the local air humidity and generates it as control value to the control means 100 can optionally also be incorporated at the outlet in addition to a temperature sensor ST. Hereby, it can be ensured that the relative air humidity actually remains below 100% so that there is no formation of condensation.

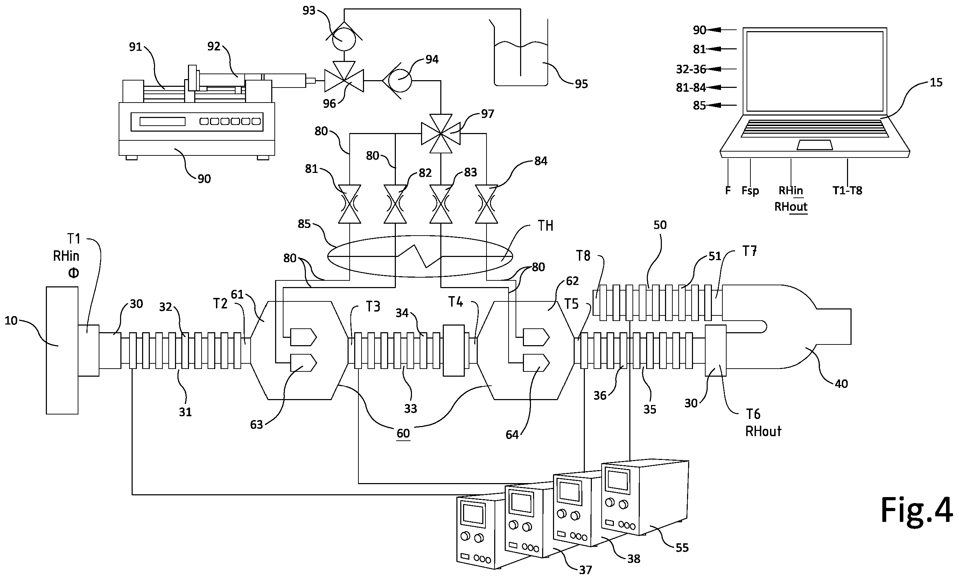

The device shown in figure 4 comprises respiratory means in the form of a standard ventilator 10 for artificial respiration or for supporting the respiration of a person involved. Such a ventilator 10 is for instance commercially available from the Drager company and, in addition to means 10 for the actual air displacement or delivery, usually also comprises a control device 15 with a user interface, whereby various settings can be set and on which a number of control parameters can be read. Particularly controllable thereby is an airflow which is supplied to a respiratory tube 30 coupled to respiratory means 10. The respiratory channel extends longitudinally in the respiratory tube 30 from a coupling to ventilator 10 to an outlet 40 with a coupling (Wye Piece) for connection to an endotracheal tube which is introduced into the patient. The coupling 40 also provides a coupling to an outgoing respiratory tube 50 whereby low-oxygen expiratory airflow can be fed back to the ventilator or otherwise. This expiratory airflow can optionally be rid of carbon dioxide and enriched with oxygen, and then recirculated to the person involved.

The respiratory tube 30 is divided into three parts, wherein each part is formed by a tube segment 31, 33, 35 of a suitable medical-grade plastic. Each tube segment 31, 33, 35 is provided with heating means in the form of a tube heating which is incorporated in the wall of the relevant tube part 31, 33, 35. The heating means in this embodiment comprise a set of spiral filaments 32, 34, 36 for each segment separately, which are each separately connected to an individually controllable electric power supply 36, 37, 38. Although the filament is drawn on the outer wall of the respiratory tube in the figure, the filament can also be incorporated in the wall or be arranged on the inner wall. Both of these have the advantage that the tube will feel less hot when touched, and the filament can thus be heated to a higher temperature. The heating of each segment 31, 33, 35 is individually controllable so that the heating means will emit in each segment an individually determined amount of heat to the airflow carried through tube 30. The outgoing expiratory tube 50 is also provided with such a spiral filament 51 and individually controllable by means of a separate electric power supply 55.

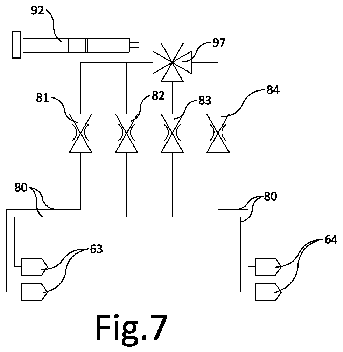

The respiratory tube 30 is provided with humidification means 61, 62 which are in each case provided between successive tube segments 31, 33, 35. Each of the humidification means comprises one or more Rayleigh atomizers 63, 64 which are coupled to respective fluid conduits 80 for supply of a humidification fluid at an increased operating pressure. In the shown device the humidification means comprise two separate spray chambers 60 in respiratory tube 30, in which the atomizers 63, 64 are accommodated. Instead, atomizers 63, 64 can also be placed directly into a respiratory tube 30 via passages provided for this purpose in the wall of the respiratory tube. Such a direct placing is shown schematically in figure 5. The atomizer 63 here forms a strong assembly with a fitting piece 70 and protrudes with interposing thereof clampingly in a passage provided for this purpose in a wall of the chamber. The fitting piece 70 has a continuous cavity 75 with fixed internal dimensions in which an atomizer body 65 with the actual spray opening(s) 66 is received. Fitting piece 70 has an outer shape and dimension adapted to the dimensions of the passage 37 provided therefor in the wall of spray chamber 60. The atomizers 63, 64 can thus be arranged and removed in equally simple manner order to be replaced with a new set of atomizers with fluid conduits, such as for instance as part of the disposable set of figure 7.

A microfilter 67 is received in cavity 75 upstream of atomizer body 65, and a microscopic preliminary filter 68 can optionally also be arranged therein. The microfilter 67 preferably comprises a ceramic plate body (chip), particularly of silicon, with therein pores (meshes) which are determined in precise and particularly in photolithographic manner and have a maximum size in the order of 3 microns. Microbes from the microscopic size of bacteria and up will be stopped thereby. A porous polymer foam can for instance be used for the preliminary filter 68.

The atomizers 63, 64 each comprise an atomizer body 65 with one or more very narrow spray channels 66 extending transversely through a spray wall of the atomizer body 65. For the atomizer body use is advantageously made of a semiconductor body, particularly a silicon chip, on which lies a thin nitride layer serving as spray wall. This material allows photolithographically very precisely determined process steps, such as optionally masked oxidation, deposition and etching processes, to be performed thereon using per se known semiconductor technology or micro-machining (MEMS) techniques, whereby the spray channels 66 can be realized on microscopic scale in highly precise manner.

In this case two types of atomizer are applied, these being distinguished by the size of these spray openings. The first atomizer(s) 63 has one or more relatively wide mutually identical spray channels with a size starting at the order of 5 microns, typically between 5 and 10 microns in diameter. The atomizer(s) 64 lying further downstream is provided with one or more mutually identical smaller spray channels, each with the same diameter of up to 5 microns, typically between 1 and 5 microns. The humidification device lying upstream thereby provides with its atomizer(s) 63 for a rough pre-humidification whereby the majority of the water vapour to be delivered is already delivered to the airflow, while with the humidifier(s) 64 lying downstream the final part of the quantity of water vapour to be supplied can be added in highly precise manner as fine-tuning for the first atomizer(s) 63.

Each atomizer 63, 64 can be drawn upon individually from the fluid conduits 80. For this purpose valves 81..84, which can be controlled individually by control device 15, are incorporated in conduits 80. In this embodiment the atomizers 63, 64 are of the Rayleigh type. A fluid for atomizing is guided under pressure via fluid conduit 80 to the relevant atomizer 63, 64. On an inlet side of the atomizer body 65 with the one or more spray channels therein this pressure is typically in the order of magnitude of 0.5 mPa to around 3 mPa. The fluid forces itself through spray opening 66 and then breaks up on an outlet side thereof into substantially identical small droplets, each having a diameter in the order of two to three times the diameter of spray opening 66. The precise droplet size in the aerosol makes the complete evaporation of the individual droplets predictable, and thereby controllable, with extraordinary precision. As alternative, so-called Savart atomizers, whereby a precise, well-defined droplet size distribution can be achieved, are optionally also suitable therefor.

For the supply of the humidification fluid the device comprises pressure means 90 in the form of a pump device 91 with a cylinder 92 in which a piston is axially movable in close-fitting manner. This is a cylinder 92 of a common medicinal syringe, of which no further requirements are per se made. The cylinder 92 lies loose in a recess provided therefor in device 91 and is thereby easily removable and exchangeable. The cylinder comprises a fluid reservoir with a volume of typically one to several millilitres. The piston body is driven by a piston rod which is coupled at a free outer end to a drive rod extending from a linear actuator of the pump device, while the syringe 92 comes up against a shoulder in the pump device on a front side.

By driving the linear actuator 91 outward in controlled manner an accurate outward stroke can be imposed on the piston body and by driving the actuator inward an inward stroke can be imposed on the piston body. During the inward stroke the cylinder draws fluid from a fluid reservoir 95 which is connected to an outer end of the cylinder. The fluid reservoir is for instance filled with pure water or a saline. Although use is in this embodiment made of a glass beaker with a riser tube, any other suitable container can be applied instead. During the outward stroke this same fluid is brought under pressure F_{SP} and guided to the fluid conduits 80. A three-way distributor 96 in combination with a set of non-return valves 93, 94 provides for the correct routing of the fluid in both stages of operation.

A multi-way distributor or hub 97 distributes the fluid over a corresponding number of fluid conduits 80 leading to the atomizers 63, 64. The fluid is supplied to the atomizers under an operating pressure of typically between 0.5 and 3 mPa. This pressure is applied by pump device 90 and cylinder 92 and is sufficient to cause a Rayleigh atomization. Depending on a force of the airflow in the inspiratory respiratory tube 30 and a fluid flow rate to be delivered therein, it is possible to draw upon either the first step 61 or the second step 62 of the humidification means, or both. The wider spray openings in the second step produce a greater fluid flow rate; the smaller openings in the first step produce smaller vapour droplets which will evaporate more easily or are entrained in the airflow in liquid form. By selectively energizing one or more of the pinch valves 81..84 the control device 15 provides for a correct dosage. In addition, control device 15 is able to have the pump device 90 produce less or more operating pressure, which likewise translates into less or more fluid flow rate through the atomizers 63, 64. An airflow and humidity sensor in the respiratory tube 30 give control device 15 the correct information and feedback for a correct and accurate control.

The control is particularly able to introduce an absolute amount of moisture into the airflow without being limited to a maximum relative humidity at the prevailing room temperature. Owing to the fine atomization of the fluid and the preheating of the airflow, the dispensed mist transitions almost immediately into the vapour form and is in this form entrained by the airflow and introduced into the respiratory system of the person involved. For this purpose the control device 15 is coupled to the heating means 32, 34, 36 and can selectively draw upon one or more of the steps therein. The separate segments 31, 33, 35 of fluid conduit 30 are each provided with temperature sensors (not further shown) whereby the temperature T₁-T₆ at the inlet and/or outlet of the relevant segment is recorded and generated to the control device 15. Also provided therein are air humidity sensors RHᵢₙ, RHₒᵤₜ and a flow sensor Φ, which are likewise coupled to control device 15. The same applies to the expiratory tube 50 in which temperature probes T₇, T₈, which generate their signals to control device 15, are also incorporated at the inlet and outlet.

The airflow is preheated by means of the heating means in order to produce at minimum the evaporation enthalpy needed for evaporation of the humidification fluid. The preheated air can furthermore contain more water vapour. A (pre-)heating of the fluid being supplied to the atomizers also contributes thereto. The feed conduits can for this purpose also be provided with heating means 85 in the form of filaments provided thereon or a sandwich construction of thermoregulatedheating plates between which the fluid conduits 80 are placed. The heating means are controlled by control device 15. A temperature sensor at fluid conduits 80 feeds back the fluid temperature T_{H}.

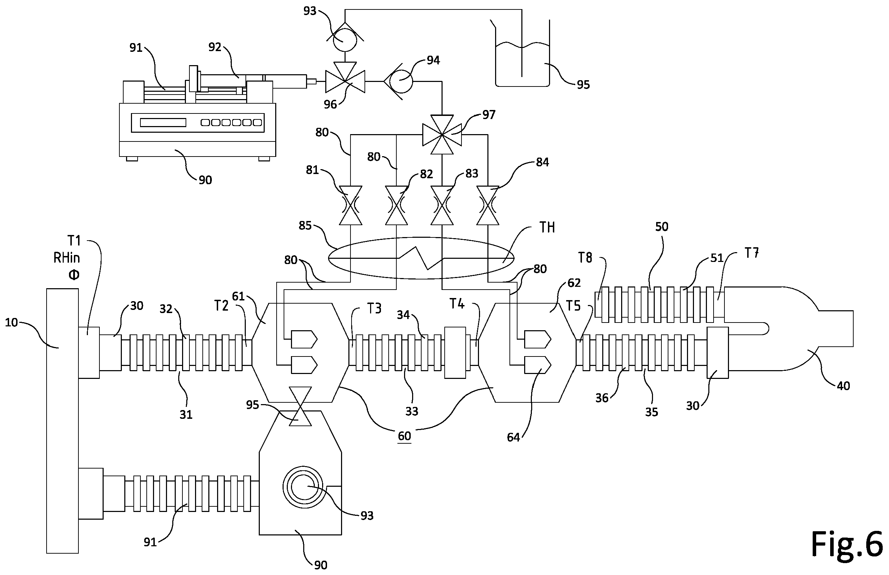

Figure 6 shows an alternative embodiment of the respiratory device according to the invention. The device is largely identical to that of figure 4, although in this case a part of the respiratory air is heated additionally in a separate heating chamber 90 which is connected via one valve 95 to the spray chamber 61. A parallel air tube 91 carries a part of the airflow from ventilator 10 to heating chamber 90 with a volume V. A heating element 93 of the heating means present therein heats this part of the air with ΔT to an increased temperature, which would be greater here than would be permitted for the main airflow being carried to the patient via respiratory tube 30. All in all, an energy Q = c.ΔT.V is thus supplied to the air in air chamber 90, wherein c represents the heat capacity per volume unit of air.

As soon as atomizer 61 is drawn upon to atomize fluid from the fluid conduit in the spray chamber the valve 95 is opened and the thus preheated air is let into the spray chamber in order to there mix with the airflow through respiratory tube 30. The preheating of the air in heating chamber 90 and of the fluid in fluid conduit 80 are here adapted such that the airflow 30 will always leave the system at a safe temperature of for instance below 40°C, but that the enthalpy Q required for evaporation of the atomized fluid is supplied at least largely, if not wholly, to the fluid for atomizing itself by means of the heating means 85 in the fluid conduit and the energy Q = c.ΔT.V which is carried into the spray chamber by the volume V of heated air. These measures together are conducive to a full evaporation of the supplied fluid in spray chamber 61 and respiratory tube 30.

The described device is able to supply a respiratory airflow in the order of 2 to 80 litres per minute and to dispense a precisely determined quantity of fluid therein. At said operating pressure a controllable fluid delivery is achievable of between 0.2 millilitres per minute to 6 millilitres per minute in a common respiratory airflow. The liquid-carrying part of the device is wholly removable and thereby easily exchangeable. In figure 7 this part is shown separately in the form of a disposable set of parts which are renewed for each patient. There is thereby always a fresh, sterile supply of fluid into the airways. A second syringe can optionally be coupled to distributor 97, whereby a medicinal fluid can simultaneously be delivered to the inspiratory airflow with an atomizer provided specifically for this purpose and having one or more spray openings adapted thereto in respect of dimension. This supply can here be dosed manually or likewise be realized with an electronically controllable metering pump provided for this purpose.

All in all, the invention provides a respiratory device with particularly practical and effective humidification means whereby a precisely determined absolute fluid delivery to the inspiratory airflow is controllable. Although the invention has been further elucidated above with reference to only a single exemplary embodiment, it will be apparent that the invention is by no means limited thereto. On the contrary, many variations and embodiments are still possible within the scope of the invention for a person with ordinary skill in the art.

## Claims

1. Respiratory device, comprising artificial respiration means (10) for generating and maintaining an oxygen-containing respiratory gas flow; a respiratory tube (30) in which a respiratory channel (35) extends for the purpose of carrying the respiratory gas flow to an outlet for supply of the respiratory gas flow for the respiratory system of a patient, which respiratory tube is coupled or at least couplable to the respiratory means (10); humidification means (61, 62) coupled or at least couplable to the respiratory tube for the purpose of humidifying the respiratory gas flow in the respiratory channel (35) with a humidification fluid; and a humidification fluid source (95) which is coupled or at least couplable via a fluid conduit to the humidification means for the purpose of providing the humidification fluid thereto, the humidification means (61, 62) comprise an atomizer (63, 64) which is able and configured to form a fine mist when the humidification fluid is excited and to deliver the fine mist on an outlet side, the atomizer (63, 64) is in open communication on the outlet side with the respiratory channel (35) in the respiratory tube (30); **characterised by** that heating means (32) are provided which enter into heat-exchanging contact with the respiratory gas flow upstream of the atomizer in order to dissipate heat thereto, and that at least the atomizer and the heating means are controlled by electronic control means configured to mutually adapt a heat dissipation by the heating means in the respiratory gas flow and a downstream delivery of the fine mist by the atomizer such that said heat dissipation suffices to make the mist formed by the atomizer transition substantially wholly, and particularly wholly, into a vapour phase when delivered by the atomizer.

2. Respiratory device according to claim 1, **characterized in that** humidification fluid is fed to the atomizer by electronically controllable dosing means, which dosing means are controlled by the control means.

3. Respiratory device according to claim 2, **characterized in that** the dosing means comprise an electronically controllable metering pump which feeds the atomizer.

4. Respiratory device according to claim 2 or 3, **characterized in that** the dosing means comprise an electronically controllable valve device between the atomizer and the humidification fluid source.

5. Respiratory device according to one or more of the preceding claims, **characterized in that** upstream of the heating means at least one sensor from a group of an air humidity sensor, a temperature sensor and a flow sensor is in operative contact with the respiratory gas flow, which at least one sensor is coupled to the control means for generating a control value thereto.

6. Respiratory device according to one or more of the preceding claims, **characterized in that** downstream of the humidification means at least one sensor from a group of an air humidity sensor, a temperature sensor and a flow sensor is in operative contact with the respiratory gas flow, which at least one sensor is coupled to the control means for generating a control value thereto.

7. Respiratory device according to one or more of the preceding claims, **characterized in that** close to the outlet at least one sensor from a group of an air humidity sensor, a temperature sensor and a flow sensor is in operative contact with the respiratory gas flow, which at least one sensor is coupled to the control means for generating a control value thereto.

8. Respiratory device according to claim 7, **characterized in that** the control means control the heating means and the humidification means in mutual relation such that the respiratory gas flow has a temperature close to the outlet which is above a dew point of the respiratory gas flow and below a maximum temperature which is still safe for the patient's respiratory system, particularly a temperature that is or is close to body temperature.

9. Respiratory device according to one or more of the preceding claims, **characterized in that** the humidification means comprise a Rayleigh type atomizer, comprising an atomizer body with a spray wall in which at least one microscopic spray opening extends over a whole thickness thereof, which at least one microscopic spray opening provides a passage between the inlet side and the outlet side of the atomizer and receives the humidification fluid under an increased operating pressure on the inlet side in order to dispense a jet thereof of mutually at least substantially identical successive liquid droplets of at least substantially a determined first size on the outlet side, and that pressure means are provided which impose the increased operating pressure on the humidification fluid and guide the humidification fluid to the atomizer under the increased operating pressure.

10. Respiratory device according to claim 9, **characterized in that** the humidification means comprise at least one similar further atomizer of the Rayleigh type with at least one further microscopic spray opening which receives the humidification fluid under an increased operating pressure on the inlet side in order to dispense a jet thereof of mutually at least substantially identical successive liquid droplets of at least substantially a determined further size on the outlet side.

11. Respiratory device according to claim 10, **characterized in that** each of the at least one spray opening of the first atomizer has a first fluid-transporting cross-section, that each of the at least one spray opening of the further atomizer has a further fluid-transporting cross-section, which further fluid-transporting cross-section differs from the first fluid-transporting cross-section.

12. Respiratory device according to claim 9, 10 or 11, **characterized in that** the humidification means comprise at least one similar further atomizer of the Rayleigh type with at least one further microscopic spray opening which receives the humidification fluid under an increased operating pressure on the inlet side in order to dispense a jet thereof of mutually at least substantially identical successive liquid droplets on the outlet side, which at least one further spray opening differs in number thereof from the at least one first microscopic spray opening of the first atomizer.

13. Respiratory device according to one or more of the preceding claims, **characterized in that** at least one further atomizer is provided, which is fed by a fluid source of a further fluid and is able and configured to form a further fine mist from the further fluid and deliver it in the respiratory channel, wherein the further fluid contains a pharmaceutically active substance.

14. Respiratory device according to claim 13, **characterized in that** the further atomizer and the humidification means are controlled and regulated in mutual relation by shared control means.

15. Respiratory device according to one or more of the preceding claims, **characterized in that** the respiratory tube is bounded at least locally by a wall and comprises in the wall a wall opening in which an atomizer is fittingly received.

16. Respiratory device according to claim 15, **characterized in that** the atomizer is placed into the wall opening via a fitting piece.

17. Respiratory device according to claim 16, **characterized in that** the atomizer and the fitting piece together form an assembly which fits releasably in the wall opening as stand-alone component.

18. Respiratory device according to claim 17, **characterized in that** the fitting piece comprises upstream of the atomizer a microscopic preliminary filter, particularly a microbial preliminary filter with maximal microscopic meshes smaller than 0.5 to 3 micrometres.

19. Respiratory device according to claim 2, **characterized in that** the dosing means comprise a container with a container volume in which a piston is movable in close-fitting manner along a first stroke and a second stroke, wherein the humidification fluid is taken from the humidification fluid source in the first stroke and is forced via the fluid conduit to the atomizer under the increased pressure in the second stroke.

20. Respiratory device according to claim 19, **characterized in that** the container comprises a combined inlet and outlet, wherein the first stroke and the second stroke comprise a mutually opposing inward and outward stroke of the piston, and wherein the combined inlet and outlet is coupled via non-return valves with opposite orientation to both respectively the humidification fluid source and the fluid conduit.

21. Respiratory device according to claim 19 or 20, **characterized in that** the container is provided with an electronically energizable actuator, particularly a linear actuator, which is coupled operatively to the piston and is controlled by the control means.

22. Respiratory device according to claim 19, 20 or 21, **characterized in that** the atomizer comprises a reservoir of a common clinical syringe.

23. Respiratory device according to claim 22, **characterized in that** the container together with the atomizer form part of a releasable disposable assembly, wherein the container is coupled to the atomizer via a fluid tube.

24. Respiratory device according to one or more of the preceding claims, **characterized in that** the respiratory tube is provided downstream of the humidification means with further heating means which are intended and configured to enter into heat-exchanging contact with a wall of the respiratory tube and particularly comprise a filament which is incorporated into the wall of the respiratory tube over a length.

25. Respiratory device according to one or more of the preceding claims, **characterized in that** the fluid conduit is provided upstream of the atomizer with electrically energizable heating means intended and configured to enter into heat-exchanging contact with the humidification fluid.

26. Respiratory device according to one or more of the preceding claims, **characterized in that** an air chamber debouches at the position of the atomizer in the respiratory channel and that the air chamber is provided with heating means for bringing a volume of air held therein to an increased temperature, and that provided between the air chamber and the respiratory channel are valve means which are connected operatively to the control means for letting in the volume of air of increased temperature at least substantially simultaneously to the fine mist.

## Patentansprüche

1. Beatmungsgerät umfassend eine künstliche Beatmungseinrichtung (10) zum Erzeugen und Aufrechterhalten eines sauerstoffhaltigen Atemgasstroms; einen Beatmungsschlauch (30), in dem sich ein Beatmungskanal (35) erstreckt, um den Atemgasstrom zu einem Auslass für die Zufuhr des Atemgasstroms für das Atmungssystem eines Patienten zu führen, wobei der Beatmungsschlauch mit der Beatmungseinrichtung (10) gekoppelt oder zumindest koppelbar ist; eine Befeuchtungseinrichtung (61, 62), die mit dem Beatmungsschlauch gekoppelt oder zumindest koppelbar ist, um den Atemgasstrom im Beatmungskanal (35) mit einem Befeuchtungsfluid zu befeuchten und eine Befeuchtungsfluidquelle (95), die über eine Fluidleitung mit der Befeuchtungseinrichtung gekoppelt oder zumindest koppelbar ist, um dort das Befeuchtungsfluid bereitzustellen, wobei die Befeuchtungseinrichtung (61, 62) einen Zerstäuber (63, 64) umfasst, der in der Lage und konfiguriert ist, einen feinen Nebel zu bilden, wenn das Befeuchtungsfluid angeregt wird, und den feinen Nebel an einer Auslassseite abzugeben, wobei der Zerstäuber (63, 64) an der Auslassseite in offener Verbindung mit dem Beatmungskanal (35) in dem Beatmungsschlauch (30) steht; **dadurch gekennzeichnet, dass** Heizmittel (32) vorgesehen sind, die stromaufwärts des Zerstäubers in wärmeaustauschenden Kontakt mit dem Atemgasstrom treten, um Wärme an diesen abzugeben, und dass zumindest der Zerstäuber und die Heizmittel durch elektronische Steuermittel gesteuert werden, die so konfiguriert sind, dass sie eine Wärmeabgabe durch die Heizmittel in den Atemgasstrom und eine stromabwärts erfolgende Abgabe des feinen Nebels durch den Zerstäuber gegenseitig so anpassen, dass die Wärmeabgabe ausreicht, um den durch den Zerstäuber gebildeten Nebel im Wesentlichen vollständig und insbesondere vollständig in eine Dampfphase übergehen zu lassen, wenn er durch den Zerstäuber abgegeben wird.

2. Beatmungsgerät nach Anspruch 1, **dadurch gekennzeichnet, dass** das Befeuchtungsfluid dem Zerstäuber durch elektronisch steuerbare Dosiermittel zugeführt wird, wobei die Dosiermittel durch die Steuermittel gesteuert werden.

3. Beatmungsgerät nach Anspruch 2, **dadurch gekennzeichnet, dass** die Dosiermittel eine elektronisch steuerbare Dosierpumpe umfassen, die den Zerstäuber speist.

4. Beatmungsgerät nach Anspruch 2 oder 3, **dadurch gekennzeichnet, dass** die Dosiermittel eine elektronisch steuerbare Ventilvorrichtung zwischen dem Zerstäuber und der Befeuchtungsfluidquelle umfassen.

5. Beatmungsgerät nach einem oder mehreren der vorhergehenden Ansprüche, **dadurch gekennzeichnet, dass** stromaufwärts der Heizeinrichtung mindestens ein Sensor aus einer Gruppe eines Luftfeuchtigkeitssensors, eines Temperatursensors und eines Strömungssensors mit dem Atemgasstrom in Wirkverbindung steht, der mit den Steuermitteln zur Erzeugung einer Steuergröße an diese gekoppelt ist.

6. Beatmungsgerät nach einem oder mehreren der vorhergehenden Ansprüche, **dadurch gekennzeichnet, dass** stromabwärts der Befeuchtungseinrichtung mindestens ein Sensor aus einer Gruppe eines Luftfeuchtigkeitssensors, eines Temperatursensors und eines Strömungssensors mit dem Atemgasstrom in Wirkverbindung steht, der mit den Steuermitteln zur Erzeugung einer Steuergröße an diese gekoppelt ist.

7. Beatmungsgerät nach einem oder mehreren der vorhergehenden Ansprüche, **dadurch gekennzeichnet, dass** in der Nähe des Auslasses mindestens ein Sensor aus einer Gruppe eines Luftfeuchtigkeitssensors, eines Temperatursensors und eines Strömungssensors mit dem Atemgasstrom in Wirkverbindung steht, der mit den Steuermitteln zur Erzeugung eines Steuerwertes gekoppelt ist.

8. Beatmungsgerät nach Anspruch 7, **dadurch gekennzeichnet, dass** die Steuermittel die Heizmittel und die Befeuchtungsmittel in gegenseitiger Beziehung so steuern, dass der Atemgasstrom nahe dem Auslass eine Temperatur aufweist, die über einem Taupunkt des Atemgasstroms und unter einer maximalen Temperatur liegt, die für das Atmungssystem des Patienten noch sicher ist, insbesondere eine Temperatur, die der Körpertemperatur entspricht oder ihr nahe kommt.

9. Beatmungsgerät nach einem oder mehreren der vorhergehenden Ansprüche, **dadurch gekennzeichnet, dass** die Befeuchtungsmittel einen Rayleigh-Typ-Zerstäuber umfassen, der einen Zerstäuberkörper mit einer Sprühwand aufweist, in der sich mindestens eine mikroskopische Sprühöffnung über deren gesamte Dicke erstreckt, wobei die mindestens eine mikroskopische Sprühöffnung einen Durchgang zwischen der Einlassseite und der Auslassseite des Zerstäubers bildet und das Befeuchtungsfluid unter einem erhöhten Betriebsdruck auf der Einlassseite aufnimmt, um einen Strahl davon aus gegenseitig mindestens im Wesentlichen identischen aufeinanderfolgenden Flüssigkeitströpfchen von mindestens im Wesentlichen einer bestimmten ersten Größe auf der Auslassseite abzugeben, und dass Druckmittel vorgesehen sind, die den erhöhten Betriebsdruck auf das Befeuchtungsfluid ausüben und das Befeuchtungsfluid unter dem erhöhten Betriebsdruck zum Zerstäuber führen.

10. Beatmungsgerät nach Anspruch 9, **dadurch gekennzeichnet, dass** die Befeuchtungsmittel mindestens einen weiteren gleichartigen Zerstäuber vom Rayleigh-Typ mit mindestens einer weiteren mikroskopischen Sprühöffnung umfassen, die das Befeuchtungsfluid unter einem erhöhten Betriebsdruck auf der Einlassseite aufnimmt, um einen Strahl davon aus gegenseitig zumindest im Wesentlichen identischen aufeinanderfolgenden Flüssigkeitströpfchen von zumindest im Wesentlichen einer bestimmten weiteren Größe auf der Auslassseite abzugeben.

11. Beatmungsgerät nach Anspruch 10, **dadurch gekennzeichnet, dass** jede der mindestens einen Sprühöffnung des ersten Zerstäubers einen ersten fluidführenden Querschnitt aufweist, dass jede der mindestens einen Sprühöffnung des weiteren Zerstäubers einen weiteren fluidführenden Querschnitt aufweist, welcher weitere fluidführenden Querschnitt sich von dem ersten fluidführenden Querschnitt unterscheidet.

12. Beatmungsgerät nach Anspruch 9, 10 oder 11, **dadurch gekennzeichnet, dass** die Befeuchtungsmittel mindestens einen ähnlichen weiteren Zerstäuber vom Rayleigh-Typ mit mindestens einer weiteren mikroskopischen Sprühöffnung umfassen, die das Befeuchtungsfluid unter einem erhöhten Betriebsdruck auf der Einlassseite empfängt, um einen Strahl davon aus gegenseitig zumindest im Wesentlichen identischen aufeinanderfolgenden Flüssigkeitströpfchen auf der Auslassseite abzugeben, wobei sich die mindestens eine weitere Sprühöffnung in ihrer Anzahl von der mindestens einen ersten mikroskopischen Sprühöffnung des ersten Zerstäubers unterscheidet.

13. Beatmungsgerät nach einem oder mehreren der vorhergehenden Ansprüche, **dadurch gekennzeichnet, dass** mindestens ein weiterer Zerstäuber vorgesehen ist, der von einer Fluidquelle eines weiteren Fluids gespeist wird und in der Lage und ausgebildet ist, aus dem weiteren Fluid einen weiteren feinen Nebel zu bilden und diesen in den Beatmungskanal abzugeben, wobei das weitere Fluid einen pharmazeutisch aktiven Wirkstoff enthält.

14. Beatmungsgerät nach Anspruch 13, **dadurch gekennzeichnet, dass** der weitere Zerstäuber und die Befeuchtungseinrichtung in gegenseitiger Beziehung durch gemeinsame Steuermittel gesteuert und geregelt werden.

15. Beatmungsgerät nach einem oder mehreren der vorhergehenden Ansprüche, **dadurch gekennzeichnet, dass** der Beatmungsschlauch zumindest lokal durch eine Wand begrenzt ist und in der Wand eine Wandöffnung aufweist, in der ein Zerstäuber passend aufgenommen ist.

16. Beatmungsgerät nach Anspruch 15, **dadurch gekennzeichnet, dass** der Zerstäuber über ein Passstück in die Wandöffnung eingesetzt ist.

17. Beatmungsgerät nach Anspruch 16, **dadurch gekennzeichnet, dass** der Zerstäuber und das Passstück zusammen eine Baugruppe bilden, die als eigenständiges Bauteil lösbar in die Wandöffnung passt.

18. Beatmungsgerät nach Anspruch 17, **dadurch gekennzeichnet, dass** das Anschlussstück stromaufwärts des Zerstäubers einen mikroskopischen Vorfilter, insbesondere einen mikrobiellen Vorfilter mit maximalen mikroskopischen Maschen von weniger als 0,5 bis 3 Mikrometern aufweist.

19. Beatmungsgerät nach Anspruch 2, **dadurch gekennzeichnet, dass** die Dosiermittel einen Behälter mit einem Behältervolumen umfassen, in dem ein Kolben entlang eines ersten Hubs und eines zweiten Hubs eng anliegend beweglich ist, wobei das Befeuchtungsfluid im ersten Hub der Befeuchtungsfluidquelle entnommen und im zweiten Hub unter dem erhöhten Druck über die Fluidleitung zum Zerstäuber gedrückt wird.

20. Beatmungsgerät nach Anspruch 19, **dadurch gekennzeichnet, dass** der Behälter einen kombinierten Einlass und Auslass umfasst, wobei der erste Hub und der zweite Hub einen einander entgegengesetzten Einwärts- und Auswärtshub des Kolbens umfassen und wobei der kombinierte Einlass und Auslass über Rückschlagventile mit entgegengesetzter Ausrichtung sowohl mit der Befeuchtungsfluidquelle als auch mit der Fluidleitung verbunden ist.

21. Beatmungsgerät nach Anspruch 19 oder 20, **dadurch gekennzeichnet, dass** der Behälter mit einem elektronisch erregbaren Aktuator, insbesondere einem Linearaktuator, versehen ist, der mit dem Kolben wirkverbunden ist und von den Steuermitteln gesteuert wird.

22. Beatmungsgerät nach Anspruch 19, 20 oder 21, **dadurch gekennzeichnet, dass** der Zerstäuber ein Reservoir einer üblichen klinischen Spritze umfasst.

23. Beatmungsgerät nach Anspruch 22, **dadurch gekennzeichnet, dass** der Behälter zusammen mit dem Zerstäuber Teil einer lösbaren Einwegbaugruppe ist, wobei der Behälter über einen Fluidschlauch mit dem Zerstäuber verbunden ist.

24. Beatmungsgerät nach einem oder mehreren der vorhergehenden Ansprüche, **dadurch gekennzeichnet, dass** der Beatmungsschlauch stromabwärts der Befeuchtungseinrichtung mit weiteren Heizmitteln versehen ist, die dazu bestimmt und ausgebildet sind, mit einer Wandung des Beatmungsschlauchs in wärmeaustauschenden Kontakt zu treten und insbesondere einen Faden umfassen, der über eine Länge in die Wandung des Beatmungsschlauchs eingearbeitet ist.

25. Beatmungsgerät nach einem oder mehreren der vorhergehenden Ansprüche, **dadurch gekennzeichnet, dass** die Fluidleitung stromaufwärts des Zerstäubers mit elektrisch erregbaren Heizmitteln versehen ist, die dazu bestimmt und ausgebildet sind, mit dem Befeuchtungsfluid in wärmeaustauschenden Kontakt zu treten.

26. Beatmungsgerät nach einem oder mehreren der vorhergehenden Ansprüche, **dadurch gekennzeichnet, dass** an der Stelle des Zerstäubers im Beatmungskanal eine Luftkammer mündet und dass die Luftkammer mit Heizmitteln versehen ist, um ein darin befindliches Luftvolumen auf eine erhöhte Temperatur zu bringen, und dass zwischen der Luftkammer und dem Beatmungskanal Ventilmittel vorgesehen sind, die mit den Steuermitteln in Wirkverbindung stehen, um das Luftvolumen mit erhöhter Temperatur zumindest im wesentlichen gleichzeitig zum feinen Nebel einzulassen.

## Revendications

1. Appareil respiratoire comprenant des moyens de respiration artificielle (10) pour générer et maintenir un flux de gaz respiratoire contenant de l'oxygène ; un tube respiratoire sur lequel s'étend un canal respiratoire (35) afin d'acheminer le flux de gaz respiratoire vers une sortie destinée à alimenter le système respiratoire d'un patient, ce tube respiratoire étant couplé ou au moins couplable aux moyens respiratoires (10) ; des moyens d'humidification (61, 62) couplés ou au moins couplables au tube respiratoire afin d'humidifier le flux de gaz respiratoire dans le canal respiratoire (35) à l'aide d'un fluide d'humidification ; et une source de fluide d'humidification (95) qui est couplée ou au moins couplable via un conduit de fluide aux moyens d'humidification dans le but de fournir le fluide d'humidification, les moyens d'humidification (61, 62) comprenant un atomiseur (63, 64) qui est capable et configuré pour former un brouillard fin lorsque le fluide d'humidification est excité et pour délivrer le brouillard fin sur un côté de sortie ; l'atomiseur (63, 64) est en communication ouverte du côté de la sortie avec le canal respiratoire (35) dans le tube respiratoire (30) ; **caractérisé par le fait que** des moyens de chauffage (32) sont prévus pour entrer en contact avec le flux de gaz respiratoire en amont de l'atomiseur afin d'en dissiper la chaleur, et qu'au moins l'atomiseur et les moyens de chauffage sont commandés par des moyens de commande électroniques configurés pour adapter mutuellement une dissipation de chaleur par les moyens de chauffage dans le flux de gaz respiratoire et une distribution en aval du brouillard fin par l'atomiseur de telle sorte que ladite dissipation de chaleur suffise à faire passer le brouillard formé par l'atomiseur pratiquement entièrement, et en particulier entièrement, en phase vapeur lorsqu'il est distribué par l'atomiseur.

2. Appareil respiratoire selon la revendication 1, **caractérisé par le fait que** le fluide d'humidification est envoyé à l'atomiseur par des moyens de dosage contrôlables électroniquement, ces moyens de dosage étant contrôlés par les moyens de commande.

3. Appareil respiratoire selon la revendication 2, **caractérisé par le fait que** les moyens de dosage comprennent une pompe doseuse à commande électronique qui permet d'alimenter l'atomiseur.

4. Appareil respiratoire selon la revendication 2 ou 3, **caractérisé par le fait que** les moyens de dosage comprennent un dispositif de valve contrôlable électroniquement entre l'atomiseur et la source de fluide d'humidification.

5. Appareil respiratoire selon l'une ou plusieurs des revendications précédentes, **caractérisé par le fait qu'**en amont du moyen de chauffage, au moins un capteur appartenant au groupe des capteurs d'humidité de l'air, de température et de débit est en contact opérationnel avec le flux de gaz respiratoire, lequel au moins un capteur est couplé au moyen de commande pour générer une valeur de commande.

6. Appareil respiratoire selon l'une ou plusieurs des revendications précédentes, **caractérisé par le fait qu'**en avai du moyens d'humidification, au moins un capteur appartenant au groupe des capteurs d'humidité de l'air, de température et de débit est en contact opérationnel avec le flux de gaz respiratoire, lequel au moins un capteur est couplé au moyen de commande pour générer une valeur de commande.

7. Appareil respiratoire selon l'une ou plusieurs des revendications précédentes, **caractérisé par le fait qu'**à proximité de la sortie, au moins un capteur appartenant au groupe des capteurs d'humidité de l'air, de température et de débit est en contact opérationnel avec le flux de gaz respiratoire, au moins un capteur étant couplé au moyen de commande pour générer une valeur de commande.

8. Appareil respiratoire selon la revendication 7, **caractérisé par le fait que** les moyens de commande permettent de contrôler les moyens de chauffage et les moyens d'humidification en relation mutuelle de manière à ce que le flux de gaz respiratoire ait une température proche de la sortie qui soit supérieure à un point de rosée du flux de gaz respiratoire et inférieure à une température maximale qui reste sans danger pour le système respiratoire du patient, en particulier une température qui est ou est proche de la température corporelle.

9. Appareil respiratoire selon l'une ou plusieurs des revendications précédentes, **caractérisé par le fait que** les moyens d'humidification comprennent un atomiseur de type Rayleigh, comprenant un corps d'atomiseur avec une paroi de pulvérisation au moins une ouverture de pulvérisation microscopique s'étendant sur toute son épaisseur, au moins une ouverture de pulvérisation microscopique fournissant un passage entre le côté entrée et le côté sortie de l'atomiseur et recevant le fluide d'humidification sous une pression de fonctionnement accrue du côté entrée afin d'en distribuer un jet de gouttelettes de liquide successives mutuellement au moins sensiblement identiques d'au moins sensiblement une première taille déterminée du côté sortie, et **par le fait que** des moyens de pression sont prévus pour imposer la pression de fonctionnement accrue au fluide d'humidification et guider le fluide d'humidification vers l'atomiseur sous la pression de fonctionnement accrue.

10. Appareil respiratoire selon la revendication 9, **caractérisé par le fait que** les moyens d'humidification comprennent au moins un autre atomiseur similaire du type Rayleigh avec au moins une autre ouverture de pulvérisation microscopique qui reçoit le fluide d'humidification sous une pression de fonctionnement accrue du côté de l'entrée afin de distribuer un jet de gouttelettes liquides successives mutuellement au moins substantiellement identiques d'au moins substantiellement une autre taille déterminée du côté de la sortie.

11. Appareil respiratoire selon la revendication 10, **caractérisé par le fait que** chacune des au moins une ouverture de pulvérisation du premier atomiseur possède une première section transversale de transport de fluide, que chacune des au moins une ouverture de pulvérisation de l'autre atomiseur possède une autre section transversale de transport de fluide, la section transversale de transport de fluide différant de la première section transversale de transport de fluide.

12. Appareil respiratoire selon la revendication 9, 10 ou 11 **caractérisé par le fait que** les moyens d'humidification comprennent au moins un autre atomiseur similaire du type Rayleigh avec au moins une autre ouverture de pulvérisation microscopique qui reçoit le fluide d'humidification sous une pression de fonctionnement accrue du côté de l'entrée afin de distribuer un jet de gouttelettes de liquide successives mutuellement au moins substantiellement identiques du côté de la sortie, dont au moins une autre ouverture de pulvérisation diffère en nombre de l'au moins une première ouverture de pulvérisation microscopique du premier atomiseur.

13. Appareil respiratoire selon l'une ou plusieurs des revendications précédentes, **caractérisé par** la présence d'au moins un autre atomiseur alimenté par une source d'un autre fluide et capable et configuré pour former un autre brouillard fin à partir de l'autre fluide et le délivrer dans le canal respiratoire, l'autre fluide contenant une substance pharmaceutiquement active.

14. Appareil respiratoire selon la revendication 13, **caractérisé par le fait que** l'atomiseur supplémentaire et le moyen d'humidification sont contrôlés et régulés en relation mutuelle par des moyens de contrôle partagés.

15. Appareil respiratoire selon l'une ou plusieurs des revendications précédentes, **caractérisé par le fait que** le tube respiratoire est délimité au moins localement par une paroi et comprend dans la paroi une ouverture dans laquelle un atomiseur est logé de manière appropriée.

16. Appareil respiratoire selon la revendication 15, **caractérisé par le fait que** l'atomiseur est placé dans l'ouverture de la paroi par l'intermédiaire d'une pièce de fixation.

17. Appareil respiratoire selon la revendication 16, **caractérisé par le fait que** l'atomiseur et la pièce de fixation forment ensemble un assemblage qui s'insère de manière amovible dans l'ouverture de la paroi en tant que composant autonome.

18. Appareil respiratoire selon la revendication 17, **caractérisé par le fait que** la pièce de fixation comporte en amont de l'atomiseur un filtre préliminaire microscopique, notamment un filtre préliminaire microbien à mailles microscopiques maximales inférieures à 0,5 à 3 micromètres.

19. Appareil respiratoire selon la revendication 2, **caractérisé par le fait que** les moyens de dosage comprennent un récipient avec un volume de récipient dans lequel un piston est déplacé de manière rapprochée le long d'une première course et d'une deuxième course, le fluide d'humidification étant prélevé de la source de fluide d'humidification dans la première course et étant forcé par le conduit de fluide vers l'atomiseur sous la pression accrue dans la deuxième course.

20. Appareil respiratoire selon la revendication 19, **caractérisé par le fait que** le récipient comprend une entrée et une sortie combinées, la première course et la deuxième course comprenant une course vers l'intérieur et vers l'extérieur mutuellement opposée du piston, et l'entrée et la sortie combinées étant couplées par des clapets anti-retour avec une orientation opposée à la fois respectivement à la source de fluide d'humidification et à la conduite de fluide.

21. Appareil respiratoire selon la revendication 19 ou 20, **caractérisé par le fait que** le récipient est pourvu d'un actionneur à énergie électronique, en particulier un actionneur linéaire, qui est couplé de manière opérationnelle au piston et est commandé par les moyens de commande.

22. Appareil respiratoire selon la revendication 19, 20 ou 21, **caractérisé par le fait que** l'atomiseur comprend un réservoir d'une seringue clinique commune.

23. Appareil respiratoire selon la revendication 22, **caractérisé par le fait que** le récipient et l'atomiseur font partie d'un ensemble jetable libérable, le récipient étant couplé à l'atomiseur par l'intermédiaire d'un tube de fluide.

24. Appareil respiratoire selon l'une ou plusieurs des revendications précédentes, **caractérisé par le fait que** le tube respiratoire est pourvu, en aval des moyens d'humidification, d'autres moyens de chauffage qui sont destinés et configurés pour entrer en contact par échange de chaleur avec une paroi du tube respiratoire et comprennent en particulier un filament qui est intégré dans la paroi du tube respiratoire sur une longueur.

25. Appareil respiratoire selon l'une ou plusieurs des revendications précédentes, **caractérisé par le fait que** le conduit de fluide est pourvu, en amont de l'atomiseur, de moyens de chauffage alimentés électriquement, destinés et configurés pour entrer en contact avec le fluide d'humidification en vue d'un échange de chaleur.

26. Appareil respiratoire selon l'une ou plusieurs des revendications précédentes, **caractérisé par le fait qu'**une chambre à air débouche à la position de l'atomiseur dans le canal respiratoire et en par la fait que la chambre à air est pourvue de moyens de chauffage pour porter un volume d'air qu'elle contient à une température plus élevée, et **par le fait que**, entre la chambre à air et le canal respiratoire, se trouvent des moyens de soupape qui sont reliés de manière opérationnelle aux moyens de commande pour laisser entrer le volume d'air à température plus élevée au moins sensiblement en même temps que le brouillard fin.
